# FASCICULE DE BREVET EUROPEEN

(11) **EP 1 530 961 B1**
(45) Date de publication et mention de la délivrance du brevet: **05.08.2009**
(21) Numéro de dépôt: 04292497.7
(22) Date de dépôt: 21.10.2004
(51) Int. Cl.: A61K 8/04, A61Q 19/00, A61Q 1/00, A61Q 19/08

(54) **Emulsion renfermant des portions de sphères creuses organosiliconées**
Emulsion enthaltend Organosilikon-Hohlkügelchen
Emulsion containing hollow organosilicon particles

(30) Priorité: 13.11.2003 FR 0350829
(43) Date de publication de la demande: 18.05.2005
(73) Titulaire: L'ORÉAL, 75008 Paris (FR)
(72) Inventeur: Cassin, Guillaume, 91140 Villebon sur Yvette (FR)
(74) Mandataire: Leonard, Armelle

(56) Documents cités:
- EP-A- 0 254 612
- WO-A-03/020225
- FR-A- 2 816 500
- JP-A- 2003 128 788
- US-A- 5 593 680
- US-A- 5 814 311

## Description

La présente invention se rapporte à une composition sous forme d'émulsion renfermant, dans un milieu physiologiquement acceptable, des portions de sphères creuses, constituées d'un matériau organosiliconé, lesdites sphères ayant un diamètre moyen allant de 0,05 à 10 µm.

Elle se rapporte également à un procédé cosmétique pour estomper les défauts de la surface de la peau, en particulier pour réduire la brillance de la peau et/ou estomper les pores, cernes, taches, rides et/ou ridules, comprenant l'application topique sur la peau de la composition précitée.

Lorsque la lumière heurte la surface de la peau, elle se sépare en deux composantes : une composante transmise et une composante réfléchie. La lumière transmise est elle-même divisée en une composante de transmission spéculaire ou directe (selon un angle unique) et en une composante de transmission diffuse (dans toutes les directions). La lumière réfléchie est également divisée en une composante de réflexion diffuse (dans toutes les directions) et en une composante de réflexion spéculaire (selon un angle unique).

Il est connu que les matériaux pulvérulents, selon leur taille, leur forme et leur nature chimique, interfèrent avec les différentes composantes ci-dessus de la lumière et ont de ce fait la capacité de modifier l'aspect de la peau.

Les recherches se sont tournées ces dernières années vers des poudres ayant une réflectance diffuse élevée, une faible réflectance spéculaire et une forte transmittance diffuse, qui permettent notamment de réduire l'apparence des rides et ridules en réduisant la différence de luminosité entre le creux et les bords de la ride.

Ces poudres, dites à effet "soft-focus", permettent également de conférer un aspect plus mat aux peaux brillantes.

Or, l'obtention d'un effet mat de la peau est très recherché par les utilisatrices à peau mixte ou grasse, ainsi que pour les compositions cosmétiques destinées à être utilisées sous les climats chauds et humides. Les reflets provoqués par un excès de sébum à la surface de la peau sont en effet généralement considérés comme inesthétiques.

Parmi les poudres à effet soft-focus proposées dans l'art antérieur, on peut citer certains mica enrobés de matériaux inorganiques et/ou de PMMA, des microsphères de silice éventuellement enrobées, des poudre de nylon, le nitrure de bore, ou encore le talc de granulométrie (ou taille moyenne des particules) 1,8 micron.

US-5593680 décrit des compositions cosmétiques sous forme de gel pour conférer un aspect mat aux peaux contenant des sphères creuses de PMMA ou des sphères inorganiques.

Il n'en demeure pas moins qu'il subsiste le besoin de disposer de matériaux permettant de camoufler les défauts superficiels de la peau.

Or, la Demanderesse a découvert, de manière surprenante et inattendue, que certaines particules de silicone, obtenues par condensation de silanols résultant de l'hydrolyse de composés organosiliconés, permettent de satisfaire ce besoin lorsqu'elles sont formulées en émulsion.

Ces particules, qui ont la forme de portions de sphères creuses, ont déjà été décrites dans les demandes de brevet JP-2003 128 788 et JP-2000 191 789. Dans ces demandes, il est suggéré de les utiliser notamment dans des produits cosmétiques pour le visage ou dans des produits de maquillage, en particulier dans des poudres compactes fonds de teint. Pour une application au domaine du maquillage, ces particules peuvent être formulées en présence d'eau et de tensioactifs. Il n'est toutefois pas suggéré de les utiliser dans des émulsions. Or, cette galénique présente un grand intérêt, en particulier dans le domaine du soin de la peau, puisqu'elle permet l'obtention de produits ayant un toucher non gras dans lesquels les propriétés optiques mises en évidence par la Demanderesse pourront être mises à profit pour camoufler les imperfections cutanées, tandis que des actifs de soin de la peau pourront éventuellement agir simultanément sur les causes de ces imperfections.

La présente invention a donc pour objet une composition sous forme d'émulsion renfermant, dans un milieu physiologiquement acceptable, des portions de sphères creuses, constituées d'un matériau organosiliconé, lesdites sphères ayant un diamètre moyen allant de 0,05 à 10 µm.

Elle se rapporte également à un procédé cosmétique pour estomper les défauts de la surface de la peau, notamment des peaux grasses ou mixtes ou des peaux âgées, en particulier pour réduire la brillance de la peau et/ou estomper les pores, cernes, taches, rides et/ou ridules, comprenant l'application topique sur la peau de la composition précitée.

Par "émulsion", on entend, au sens de la présente invention, un système à au moins deux phases constitué de deux liquides ou plus, non miscibles ou partiellement miscibles entre eux, dont l'un -qui forme la phase dispersée- est dispersé dans l'autre -qui forme la phase continue- sous forme de fines gouttelettes, dont le diamètre n'excède généralement pas 5 microns.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau siliconé constituant ces portions de sphères creuses est de préférence un polysiloxane réticulé de structure tridimensionnelle ; il comprend de préférence, voire est constitué par, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif, un groupe organique non réactif, et de préférence un groupe organique non réactif.

Le groupe organique non réactif peut être un groupe alkyle en C₁-C₄, notamment un groupe méthyle, éthyle, propyle, butyle, ou un groupe phényle, et de préférence un groupe méthyle.

Le groupe organique réactif peut être un groupe époxy, un groupe (méth)acryloxy, un groupe alkényle, un groupe mercaptoalkyle, aminoalkyle, ou halogénoalkyle, un groupe glycéroxy, un groupe uréïdo, ou un groupe cyano. De préférence, le groupe organique réactif peut être un groupe époxy, un groupe (méth)acryloxy, un groupe alkényle, ou un groupe mercaptoalkyle ou aminoalkyle. Le groupe organique réactif comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.
Comme groupe (méth)acryloxy, on peut citer un groupe 3-méthacryloxypropyl, un groupe 3-acryloxypropyl.
Comme groupe alkényle, on peut citer un groupe vinyl, allyl, isopropényl.
Comme groupe mercaptoalkyle, on peut citer un groupe mercaptopropyl, mercaptoéthyl.
Comme groupe aminoalkyle, on peut citer un groupe 3-(2-aminoéthyl)aminopropyl, un groupe 3-aminopropyl, un groupe N,N-diméthylaminopropyl.
Comme groupe halogénoalkyle, on peut citer un groupe 3-chloropropyl, un groupe trifluoropropyl.
Comme groupe glycéroxy, on peut citer un groupe 3-glycéroxypropyl, un groupe 2-glycéroxyéthyl.
Comme groupe uréido, on peut citer un groupe 2-uréidoéthyl.
Comme groupe cyano, on peut citer un groupe cyanopropyl ou cyanoéthyl.

De préférence, R₁ désigne un groupe méthyle.

Ces portions de sphère creuses sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (I) de formule SiX₄ et d'un composé (II) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium et a notamment la signification indiquée précédemment pour R₁ ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) à une réaction de condensation.

En ce qui concerne les groupements X et Y des composés (I) et (II), on peut utiliser :
- comme groupes alcoxy en C₁-C₄ : les groupes méthoxy, éthoxy ;
- comme groupes alkoxyéthoxy renfermant un groupe alcoxy en C₁-C₄ ; les groupes méthoxyéthoxy, butoxyéthoxy ;
- comme groupes alcoyloxy en C₂-C₄ : les groupes acétoxy, propioxy ;
- comme groupes N,N-dialkylamino renfermant un groupement alkyle en C₁-C₄ : les groupes diméthylamino, diéthylamino ;
- comme atome d'halogène :les atomes de chlore ou de brome.
-

De préférence, X et/ou Y désigne un groupe alcoxy en C₁-C₄.

Ainsi, on peut citer comme composés de formule (I) : le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (I) est choisi parmi le tétraméthoxysilane, le tétraéthoxysilane, le tétrabutoxysilane, et leurs mélanges.

On peut citer comme composés de formule (II) comportant un groupe organique R non réactif : le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, et le méthyltrihydrogénosilane.

Enfin, on peut citer comme composés de formule (II) comportant un groupe organique R réactif :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, et le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréido comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

Des exemples préférés de ce type de composés de formule (II) sont choisis parmi : les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (I) et (II) préférés pour la mise en oeuvre de cette invention sont respectivement le tétraéthoxysilane et le méthyltriméthoxysilane. Après polymérisation de ces motifs, les portions de sphères selon l'invention ont donc avantageusement une structure réticulée constituée d'unités SiO₂ et d'unités R₁SiO_{1,5} où R₁ est préférentiellement un groupe méthyle.

Dans l'étape (a), le rapport molaire du composé (I) au composé (II) va habituellement de 30:70 à 50:50, avantageusement de 35:65 à 45:55, et est préférentiellement de 40:60. Le rapport en poids de l'eau au total des composés (I) et (II) va de préférence de 10:90 à 70:30. L'ordre d'introduction des composés (I) et (II) dépend généralement de leur vitesse d'hydrolyse. La température de la réaction d'hydrolyse va habituellement de 0°C à 40°C et ne dépasse habituellement pas 30°C pour éviter une condensation prématurée des composés.

Comme catalyseurs d'hydrolyse et de polymérisation, on peut utiliser des catalyseurs basiques -tels que l'hydroxyde de sodium, le carbonate de sodium ou des amines- ou des catalyseurs acides, choisis parmi les acides organiques -tels que l'acide acétique ou le dodécylbenzènesulfonate de sodium- ou inorganiques -tels que l'acide chlorhydrique-. Lorsqu'il est présent, le tensioactif est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (I) et (II), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

Les sphères obtenues ont un diamètre moyen allant de 0,05 à 10 µm.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788.

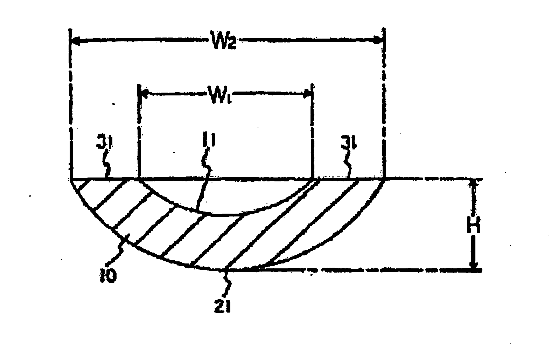

La Figure annexée illustre ce type de portions de sphères en coupe transversale.

Comme il ressort de cette Figure, ces portions de sphères sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les deux extrémités du grand arc externe (21) allant de 0,05 à 10 µm en moyenne et la hauteur (H) du grand arc externe (21) allant de 0,015 à 8 µmen moyenne. Les segments (31) ont de préférence une longueur (W2-W1)/2 (ci-après désignée par "épaisseur des particules") allant de 0,05 à 1 µm, plus préférentiellement de 0,1 à 0,8 µm.

Les dimensions mentionnées ci-dessus sont obtenues en calculant la moyenne des dimensions de cent particules choisies sur une image obtenue au microscope électronique à balayage.

Selon une forme d'exécution avantageuse de l'invention, le rapport Ra de la largeur W2 à l'épaisseur (W2-W1)/2 est au moins égal à 3, de préférence supérieur à 5 et, mieux, supérieur à 10, voire supérieur à 20. La Demanderesse a en effet découvert que plus ce rapport était élevé, meilleures étaient les propriétés optiques des matériaux selon l'invention et plus la quantité de matériau nécessaire pour obtenir un effet voulu était faible.

Comme portions de sphères utilisables selon l'invention, on peut citer les particules en forme de bol commercialisées dans la série NLK par la société TAKEMOTO OIL & FAT, qui sont constituées de l'organosilicone réticulé TAK-110 (polymère réticulé méthylsilanol/silicate) de cette même société, en particulier les particules vendues sous la dénomination NLK-506.
La quantité de portions de sphères utilisées dans la composition selon l'invention peut représenter de 0,1 à 20%, de préférence de 0,5 à 15% et, mieux, de 0,5 à 10% du poids total de la composition.

La composition selon l'invention est de préférence adaptée à une application topique sur la peau et comprend donc généralement un milieu physiologiquement acceptable, c'est-à-dire compatible avec la peau et éventuellement avec ses phanères. De préférence, il s'agit d'un milieu cosmétiquement acceptable, c'est-à-dire qui a un aspect, en particulier une couleur, une odeur et un toucher agréables et qui ne génère pas d'inconforts (rougeurs, picotements, tiraillements) susceptibles de détourner l'utilisateur de la composition.

La composition selon l'invention peut se présenter sous forme d'émulsion obtenue par dispersion d'une phase aqueuse dans une phase grasse (E/H) ou d'une phase grasse dans une phase aqueuse (H/E), de consistance liquide ou semi-liquide du type lait, ou de consistance molle, semi-solide ou solide du type crème ou gel, ou encore d'émulsion multiple (E/H/E ou H/E/H). Ces compositions sont préparées selon les méthodes usuelles.

Elle peut avoir la forme d'un produit de soin ou de maquillage du visage et/ou du corps, et être conditionnée par exemple sous forme de crème en pot ou de fluide en tube ou en flacon pompe.

Selon un mode préféré de réalisation de l'invention, la composition se présente sous forme d'une émulsion H/E.

Comme exemples d'huiles utilisables dans la composition selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam® ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Quand elle est présente, la quantité de phase huileuse peut aller par exemple de 0,01 à 50 % en poids et de préférence de 0,1 à 30 % en poids par rapport au poids total de la composition.

Selon une forme d'exécution particulièrement avantageuse de l'invention, la composition comprend au moins une huile volatile. La Demanderesse a en effet mis en évidence que ce type d'huile améliorait les propriétés optiques de la composition comprenant les portions de sphères creuses ouvertes organosiliconées décrites précédemment.

Par cette expression, on entend des huiles ayant à une température de 20°C une pression de vapeur supérieure à 1 mbar. La pression de vapeur est définie comme la pression à laquelle un liquide et sa vapeur sont en équilibre à une température donnée. On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, l'octaméthyltrisiloxane et l'hexaméthyldisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de "PF 5050®" et "PF 5060®" par la Société 3M et les dérivés de perfluoromorpholine, tels que la 4-trifluorométhyl perfluoromorpholine vendue sous la dénomination "PF 5052®" par la Société 3M.

La composition selon l'invention peut en outre contenir divers adjuvants couramment utilisés dans le domaine cosmétique, tels que des émulsionnants ; des charges ; des séquestrants ; des colorants ; des parfums ; et des épaississants et gélifiants. Les quantités de ces différents adjuvants et leur nature seront choisis de manière à ne pas nuire aux propriétés optiques de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion pouvant aller par exemple de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90^{R} par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glyceryl stearate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate), le monostéarate de polyéthylène glycol 100 OE (nom CTFA : PEG-100 stearate) et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesquiisostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic et leurs mélanges.

Comme indiqué précédemment, selon la fluidité de la composition que l'on souhaite obtenir, on peut incorporer dans la composition, un ou plusieurs gélifiants, notamment hydrophiles, c'est-à-dire solubles ou dispersibles dans l'eau.

Comme gélifiants hydrophiles, on peut citer en particulier les polymères épaississants hydrosolubles ou hydrodispersibles. Ceux-ci peuvent notamment être choisis parmi : les polymères carboxyvinyliques modifiés ou non, tels que les produits commercialisés sous les dénominations Carbopol (nom CTFA : carbomer) et Pemulen (nom CTFA : Acrylates/C10-30 akyl acrylate crosspolymer) par la société Goodrich ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose muicrocristalline, la carboxyméthylcellulose, l'hydroxyméthylcellullose et l'hydroxypropylcellulose ; et leurs mélanges.

Comme gélifiants lipophiles, on peut citer par exemple les argiles modifiées telles que le silicate de magnésium modifié (bentone gel VS38 de RHEOX), l'hectorite modifiée par le chlorure de distéaryl diméthyl ammonium (nom CTFA : Disteardimonium hectorite) commercialisé sous la dénomination « bentone 38 CE » par la société RHEOX.

Comme indiqué précédemment, la composition selon l'invention peut en outre comprendre des charges, ayant éventuellement un effet matifiant susceptible de renforcer les effets des portions de sphères selon l'invention.

Comme charges, on peut citer par exemple, les particules de polyamide (Nylon) et notamment les microbilles vendues sous les dénominations ORGASOL par la société Atochem ou les fibres de nylon ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les particules de résine mélamine-formaldéhyde ou d'urée-formaldéhyde ; les particules de poly(tétrafluoroéthylène) ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm), ou les microsphères de polyacrylonitrile commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch, ou les microbilles de cellulose ; les microbilles de résine de silicone telles que celles commercialisées sous la dénomination TOSPEARL par la société Toshiba Silicone, notamment TOSPEARL 240 ; et leurs mélanges.

D'autres exemples d'agents matifiants utilisables dans la composition selon l'invention sont les polymères comprenant des unités à LCST, tels que décrits dans la demande FR-2 838 345 ; les copolymères de vinylpyrrolidone et de 1-triacontène ; et les argiles.

Pour renforcer les effets de la composition selon l'invention, celle-ci peut en outre ou en variante renfermer un ou plusieurs actifs complétant l'effet obtenu à l'aide des portions de sphères creuses siliconées.

Pour une application en particulier au soin ou au maquillage des peaux grasses, la composition selon l'invention peut comprendre au moins un actif choisi parmi : les agents desquamants, les agents anti-séborrhéiques, les agents anti-microbiens, et les agents apaisants.

Pour une application en particulier au soin ou au maquillage des peaux âgées, la composition selon l'invention peut comprendre au moins un actif choisi parmi : les agents desquamants ou hydratants ; les agents dépigmentants ou anti-pigmentants ; les agents anti-glycation ; les agents anti-NO ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents myorelaxants ou dermo-décontractants ; les agents anti-radicalaires ou anti-pollution ; les agents tenseurs ; et les agents agissant sur la micro-circulation.

Des exemples de tels actifs seront maintenant donnés.

### 1. Agents desquamants et hydratants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.

### 2. Agent dépigmentant, anti-pigmentant ou pro-pigmentant

Les agents dépigmentants ou anti-pigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

### 3. Agent anti-glycation

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (*Vaccinium angusfifollium*); l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 2 802 425, FR 2 810 548, FR 2 796 278 et FR 2 802 420, respectivement. Le resvératrol est particulièrement préféré pour une utilisation dans cette invention.

### 4. Inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce Olea europaea qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

### 5. Agent anti-séborrhéique

Lorsque la composition selon l'invention comprend un agent anti-séborrhéique tel qu'un inhibiteur de 5α-réductase, celui-ci peut notamment être choisi parmi :
- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5®;
- un extrait de Laminaria saccharina commercialisé notamment par la société SECMA sous la dénomination commerciale Phlorogine®;
- un extrait de Spiraea ulmaria commercialisé notamment par la société SILAB sous la dénomination commerciale Sebonormine®;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED;
- des extraits de plantes du genre Silybum ;
- des extraits végétaux contenant des sapogénines et en particulier les extraits de Dioscorées riches en diosgénine ou hécogénine ; et
- des extraits d'Eugenia caryophyllata contenant de l'eugénol et du glucoside d'eugényle.

### 6. Agent stimulant la synthèse de macromolécules dermiques ou épidermiques et/ou empêchant leur dégradation

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine®; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin®; et l'extrait d'algue Macrocystis pyrifera commercialisé par la société SECMA sous la dénomination commerciale Kelpadelie®;
- soit sur la synthèse des glycosaminoglycanes, tels que le produit de fermentation du lait par lactobacillus vulgaris, commercialisé par la société BROOKS sous la dénomination commerciale Biomin yogourth®; l'extrait d'algue brune Padina pavonica commercialisé par la société ALBAN MÜLLER sous la dénomination commerciale HSP3®; et l'extrait de Saccharomyces cerevisiae disponible notamment auprès de la société SILAB sous la dénomination commerciale Firmatlft® ou auprès de la société LSN sous la dénomination commerciale Cytovitin®;
- soit sur la synthèse de la fibronectine, tels que l'extrait de zooplancton Salina commercialisé par la société SEPORGA sous la dénomination commerciale GP4G®; l'extrait de levure disponible notamment auprès de la société ALBAN MÜLLER sous la dénomination commerciale Drieline®; et le palmitoyl pentapeptide commercialisé par la société SEDERMA sous la dénomination commerciale Matrixil®;
- soit sur l'inhibition des métalloprotéinases (MMP) telles que plus particulièrement les MMP 1, 2, 3, 9. On peut citer: les rétinoïdes et dérivés, les oligopeptides et les lipopeptides, les lipoaminoacides, l'extrait de malt commercialisé par la société COLETICA sous la dénomination commerciale Collalift®; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl®; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine®; l'extrait de bourgeons de hêtre Fagus sylvatica commercialisé par la société GATTEFOSSE sous la dénomination commerciale Gatuline®; et l'extrait de zooplancton Salina commercialisé par la société SEPORGA sous la dénomination commerciale GP4G®.

### 7. Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®); et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine®; et l'extrait de maïs commercialisé par la société SOLABIA sous la dénomination commerciale Phytovityl®; et les lignanes tels que le sécoisolaricirésinol..

### 8. Agent myorelaxant ou dermo-décontractant

Les agents myorelaxants ou dermo-décontractants utilisables dans la composition selon l'invention comprennent l'alvérine et ses sels, le gluconate de manganèse, le Diazepam, l'hexapeptide Argireline R commercialisé par la société LIPOTEC, certaines amines secondaires et tertiaires carbonylées, l'adénosine, ainsi que les sapogénines et les extraits naturels, en particulier de Wild Yam, en contenant, ainsi que les extraits de Boswellia serrata.

### 9. Agent anti-microbien

Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le farnesol, les phytosphingosines et leurs mélanges.

Les agents antimicrobiens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.

### 10. Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou L021,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin,
(3) les silicates mixtes, notamment les phyllosilicates et en particulier les Laponites,
(4) les microparticules de cire, choisies par exemple parmi les cires de Carnauba, de Candelila ou d'Alfa,
(5) les particules colloïdales de charge inorganique ayant un diamètre moyen en nombre allant de 0,1 à 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

### 11. Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le coenzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

### 12. Agents apaisants

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'Echium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa monieri, les phytostérols, la cortisone, l'hydrocortisone, l'indométhacine et la beta méthasone.

### 13. Agents agissant sur la microcirculation

Les actifs agissant sur la microcirculation (vasoprotecteurs ou vasodilatateurs) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.

L'invention sera maintenant illustrée par les exemples non limitatifs suivants. Dans ces exemples, les quantités sont indiquées en pourcentage pondéral.

### EXEMPLES

### Exemple 1 : Compositions cosmétiques

| *Emulsions H*/*E* | *A (comparatif*) | *B* | *C* | *D* |
|---|---|---|---|---|
| Gomme de xanthane | 0.20% | 0.20% | 0.20% | 0.20% |
| Poly(acide 2-acrylamido 2-méthylpropane sulfonique) réticulé | 0.40% | 0.40% | 0.40% | 0.40% |
| Cyclohexasiloxane | 10.0% | 10.0% | 10.0% | 10.0% |
| Hydroxyde de sodium | 0.01% | 0.01% | 0.01% | 0.01% |
| Glycérine | 5.00% | 5.00% | 5.00% | 5.00% |
| Alcool stéarylique | 1.00% | 1.00% | 1.00% | 1.00% |
| Stéarates de glycéryle et de polyoxyéthylène (100 OE) | 2.00% | 2.00% | 2.00% | 2.00% |
| Tartarate de dimyristyle, alcool cétéarylique, alcols gras en C₁₂₋₁₅ oxyéthylénés (7 OE) et alcool laurylique oxyéthyléné (25 OE) et oxypropyléné (25 OE) | 1.50% | 1.50% | 1.50% | 1.50% |
| **Particules sphériques d'organopolysiloxane réticulé⁽¹⁾** | 5.00 % | | | |
| **Particules hémisphériques d'organopolysiloxane réticulé⁽²⁾** | | 5.00% | | |
| **Particules hémisphériques d'organopolysiloxane réticulé⁽³⁾** | | | 5.00% | |
| **Particules hémisphériques d'organopolysiloxane réticulé⁽⁴⁾** | | | | 5.00% |
| Conservateurs | 0.70% | 0.70% | 0.70% | 0,70% |
| Eau | qsp 100 % | qsp 100 % | qsp 100 % | qsp 100 % |

| | | | | |
|---|---|---|---|---|
| ⁽¹⁾Particules sphériques de diamètre 3,2 µm constituées d'un copolymère dimethiconol / silsesquioxane | | | | |
| ⁽²⁾ Particules constituées de l'organosilicone réticulé TAK-110 (polymère réticulé méthylsilanol/silicate) de la société TAKEMOTO OIL & FAT, en forme de bol, de largeur 2,5 µm, de hauteur 1,5 µm et d'épaisseur 350 nm | | | | |
| ⁽³⁾ Particules constituées de l'organosilicone réticulé TAK-110 (polymère réticulé méthylsilanol/silicate) de la société TAKEMOTO OIL & FAT, en forme de bol, de largeur 2,5 µm, de hauteur 1,5 µm et d'épaisseur 600 nm | | | | |
| ⁽⁴⁾ Particules constituées de l'organosilicone réticulé TAK-110 (polymère réticulé méthylsilanol/silicate) de la société TAKEMOTO OIL & FAT, en forme de bol, de largeur 9,5 µm, de hauteur µm et d'épaisseur 350 nm. | | | | |

Ces compositions sont préparées de manière classique pour l'homme du métier, en ajoutant, sous agitation, la phase huileuse chauffée à 65°C à la phase aqueuse chaude.

### Exemple 2 : évaluation in vitro des propriétés optiques

On a mesuré la matité obtenue avec les compositions B à D selon l'invention, et avec la composition A donné à titre d'exemple comparatif, en utilisant une carte de contraste (Prufkarte type 24/5 - 250 cm²) commercialisée par la société ERICHSEN. La composition a été étalée à raison de 2 mg/cm² à l'aide d'un tire-film mécanique. On a ensuite séché la composition pendant une nuit à une température de 37°C, puis on a mesuré la réflexion à l'aide d'un gonioréflectomètre. Le résultat obtenu est le rapport R entre la réflexion spéculaire et la réflexion diffuse. La valeur de R est d'autant plus faible que l'effet matifiant est important.

| Composition | A | B | C | D |
|---|---|---|---|---|
| R | 2,00 ± 0,09 | 0,85 ± 0,10 | 0,88 ± 0,02 | 0,55 ± 0,05 |

Ces résultats, in vitro, montrent qu'avec une concentration à 5% (matière active) de particules hémisphériques d'organopolysiloxane, on obtient un résultat de matité bien supérieur à celui obtenu avec la formule comparative contenant des particules sphériques organosiliconées.

### Exemple 3 : évaluation in vivo

L'effet matifiant, et les aspects cosmétiques, d'une formule correspondant à la composition B de l'Exemple 1, mais renfermant 8% de particules hémisphériques d'organopolysiloxane au lieu de 5%, ont été évalués sur un panel de sept femmes ayant une peau grasse. Il a été jugé que cette composition matifiait la peau et affinait son grain, les pores paraissant moins dilatés et le teint plus unifié. Un effet de "floutage" des défauts a été observé (effet soft-focus).

### Exemple 4: évaluation in vivo

On a évalué, sur un panel de six femmes ayant une peau grasse et présentant des irrégularités du micro-relief, les effets optiques sur la peau d'une composition correspondant à la composition B de l'Exemple 1, mais contenant 8% en poids de particules hémisphériques d'organopolysiloxane réticulé obtenues sous la dénomination commerciale NLK-506 auprès de Takemoto Oil and Fat.

On a observé que la composition testée matifiait instantanément les reflets de la surface de la peau et diminuait la visibilité du micro-relief en affinant le grain de peau, les pores semblant moins dilatés.

## Revendications

1. Composition sous forme d'émulsion renfermant, dans un milieu physiologiquement acceptable, des portions de sphères creuses, constituées d'un matériau organosiliconé, lesdites sphères ayant un diamètre moyen allant de 0,05 à 10 µm.

2. Composition selon la revendication 1, **caractérisée en ce que** le matériau siliconé constituant les portions de sphères creuses est un polysiloxane réticulé de structure tridimensionnelle constitué de motifs de formule (I) : SiO₂ et de formule (II) : R¹SiO₁,₅ dans lesquels R¹ désigne un groupe organique ayant un atome de carbone directement relié à l'atome de silicium.

3. Composition selon la revendication 2, **caractérisée en ce que** R¹ est un groupe organique non réactif choisi parmi : un groupe méthyle, éthyle, propyle, butyle, ou un groupe phényle.

4. Composition selon la revendication 2, **caractérisée en ce que** R¹ est un groupe organique réactif choisi parmi : un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano, comprenant de 2 à 6 atomes de carbone.

5. Composition selon la revendication 3, **caractérisée en ce que** R₁ désigne un groupe méthyle.

6. Composition selon l'une quelconque des revendications 1 à 5, **caractérisée en ce que** lesdites portions de sphères sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm en moyenne, la largeur (W2) entre les deux extrémités du grands arc externe (21) allant de 0,05 à 10 µm en moyenne et la hauteur (H) du grand arc externe (21) allant de 0,015 à 8 µm en moyenne.

7. Composition selon la revendication 6, **caractérisée en ce que** les segments (31) ont une longueur (W2-W1)/2 allant de 0,05 à 1 µm.

8. Composition selon la revendication 6 ou 7, **caractérisée en ce que** le rapport Ra de la largeur W2 à l'épaisseur (W2-W1)/2 est au moins égal à 3.

9. Composition selon la revendication 8, **caractérisée en ce que** le rapport Ra de la largeur W2 à l'épaisseur (W2-W1)/2 est supérieur à 5.

10. Composition selon la revendication 9, **caractérisée en ce que** le rapport Ra de la largeur W2 à l'épaisseur (W2-W1)/2 est supérieur à 10.

11. Composition selon la revendication 10, **caractérisée en ce que** le rapport Ra de la largeur W2 à l'épaisseur (W2-W1)/2 est supérieur à 20.

12. Composition selon l'une quelconque des revendications 2 à 11, **caractérisée en ce que** lesdites sphères sont susceptibles d'être obtenues selon un procédé comprenant :
(a) l'introduction dans un milieu aqueux, en présence d'un catalyseur d'hydrolyse, et éventuellement d'un tensioactif, d'un composé (I) de formule SiX₄ et d'un composé (II) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et .
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

13. Composition selon la revendication 12, **caractérisée en ce que** le composé de formule (I) est choisi parmi : le tétraméthoxysilane, le tétraéthoxysilane, le tétrabutoxysilane, le triméthoxyéthoxysitane, le tributoxyéthoxysilane, le tétraacétoxysilane, le tétrapropioxysilane, le tétra(diméthylamino)silane, le tétra(diéthylamino)silane, le silane tétraol, le chlorosilane triol, le dichlorodisilanol, le tétrachlorosilane, le chlorotrihydrogénosilane.

14. Composition selon la revendication 12 ou 13, **caractérisée en ce que** le composé de formule (II) comporte un groupe organique R non réactif et est choisi parmi : le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

15. Composition selon la revendication 12 ou 13, **caractérisée en ce que** le composé de formule (II) comporte un groupe organique R réactif et est choisi parmi : les silanes ayant un groupe époxy ; les silanes ayant un groupe (méth)acryloxy ; les silanes ayant un groupe alkényle ; les silanes ayant un groupe mercapto ; les silanes ayant un groupe aminoalkyle ; les silanes ayant un groupe halogénoalkyl ; les silanes ayant un groupe glycéroxy ; les silanes ayant un groupe uréido ; les silanes ayant un groupe cyano.

16. Composition selon l'une quelconque des revendication 12 à 15, **caractérisée en ce que** X et/ou Y désigne un groupe alcoxy en C₁-C₄

17. Composition selon la revendication 12 ou 16, **caractérisée en ce que** le composé (I) est le tétraéthoxysilane.

18. Composition selon la revendication 12 ou 16, **caractérisée en ce que** le composé (II) est le méthyltriméthoxysilane.

19. Composition selon l'une quelconque des revendications 12 à 15, **caractérisée en ce que**, dans l'étape (a), le rapport molaire du composé (I) au composé (II) va de 30:70 à 50:50.

20. Composition selon la revendication 19, **caractérisée en ce que** le rapport molaire du composé (I) au composé (II) va de 35:65 à 45:55.

21. Composition selon la revendication 19, **caractérisée en ce que** le rapport molaire du composé (I) au composé (II) est de 40:60.

22. Composition selon l'une quelconque des revendications 12 à 21, **caractérisée en ce que** le rapport en poids de l'eau au total des composés (I) et (II) va de 10:90 à 70:30 dans l'étape (a).

23. Composition selon l'une quelconque des revendications 12 à 22, **caractérisée en ce que** les catalyseurs d'hydrolyse et de polymérisation sont indépendamment choisis parmi l'hydroxyde de sodium, le carbonate de sodium, l'acide acétique, le dodécylbenzènesulfonate de sodium et l'acide chlorhydrique.

24. Composition selon l'une quelconque des revendications 12 à 23, **caractérisée en ce que** le tensioactif est le dodécylbenzènesulfonate de sodium.

25. Composition selon l'une quelconque des revendications 1 à 24, **caractérisée en ce que** la quantité de portions de sphères représente de 0,5 à 10% du poids total de la composition.

26. Composition selon l'une quelconque des revendications 1 à 25, **caractérisé en ce qu'**elle se présente sous la forme d'une émulsion H/E.

27. Composition selon l'une quelconque des revendications 1 à 26, **caractérisée en ce qu'**elle renferme au moins une huile volatile.

28. Composition selon la revendication 27, **caractérisée en ce que** ladite huile volatile est choisie parmi : les silicones cycliques ou linéaires renfermant de 2 à 6 atomes de silicium, les hydrocarbures ramifiés, les perfluoroalcanes volatiles et les dérivés de perfluoromorpholine.

29. Procédé cosmétique pour estomper les défauts de la surface de la peau, comprenant l'application topique sur la peau de la composition selon l'une quelconque des revendications 1 à 28.

30. Procédé selon la revendication 29, **caractérisé en ce qu'**il est destiné à réduire la brillance de la peau et/ou estomper les pores.

31. Procédé selon la revendication 29 ou 30, **caractérisé en ce que** ladite peau est une peau grasse ou mixte.

32. Procédé selon la revendication 30 ou 31, **caractérisé en ce que** la composition renferme en outre au moins un actif choisi parmi : les agents desquamants, les agents anti-séborrhéiques, les agents anti-microbiens, et les agents apaisants.

33. Procédé selon la revendication 29, **caractérisé en ce qu'**il est destiné à estomper les cernes, taches, rides et/ou ridules.

34. Procédé selon la revendication 29 ou 33, **caractérisé en ce que** ladite peau est une peau âgée.

35. Procédé selon la revendication 33 ou 34, **caractérisé en ce que** la composition renferme en outre au moins un actif choisi parmi : les agents desquamants ou hydratants ; les agents dépigmentants ou anti-pigmentants ; les agents anti-glycation ; les agents anti-NO ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents myorelaxants ou dermo-décontractants ; les agents anti-radicalaires ou anti-pollution ; les agents tenseurs ; et les agents agissant sur la microcirculation. '

## Claims

1. Composition in the form of an emulsion containing, in a physiologically acceptable medium, portions of hollow spheres, composed of an organosilicone material, said spheres having an average diameter ranging from 0.05 to 10 µm.

2. Composition according to Claim 1, **characterized in that** the silicone material constituting the hollow sphere portions is a crosslinked polysiloxane of three-dimensional structure composed of units of formula (I): SiO₂ and of formula (II): R¹SiO₁.₅ in which R¹ denotes an organic group having a carbon atom directly bonded to the silicon atom.

3. Composition according to Claim 2, **characterized in that** R¹ is a non-reactive organic group chosen from: a methyl, ethyl, propyl or butyl group, for a phenyl group.

4. Composition according to Claim 2, **characterized in that** R¹ is a reactive organic group chosen from: an epoxy group, a (meth)acryloxy group, an alkenyl group, a mercaptoalkyl, aminoalkyl or haloalkyl group, a glyceroxy group, a ureido group or a cyano group, comprising from 2 to 6 carbon atoms.

5. Composition according to Claim 3, **characterized in that** R¹ denotes a methyl group.

6. Composition according to any one of Claims 1 to 5, **characterized in that** said sphere portions are formed (in cross section) from a small inner arc (11), from a large outer arc (21) and from segments (31) which connect the ends of the respective arcs, the width (W1) between the two ends of the small inner arc (11) ranging from 0.01 to 8 µm on average, the width (W2) between the two ends of the large outer arc (21) ranging from 0.05 to 10 µm on average and the height (H) of the large outer arc (21) ranging from 0.015 to 8 µm on average.

7. Composition according to Claim 6, **characterized in that** the segments (31) have a length (W2-W1)/2 ranging from 0.05 to 1 µm.

8. Composition according to Claim 6 or 7, **characterized in that** the ratio Ra o.f the width W2 to the thickness (W2-W1)/2 is at least equal to 3.

9. Composition according to Claim 8, **characterized in that** the ratio Ra of the width W2 to the thickness (W2-W1)/2 is greater than 5.

10. Composition according to Claim 9, **characterized in that** the ratio Ra of the width W2 to the thickness (W2-W1)/2 is greater than 10.

11. Composition according to Claim 10, **characterized in that** the ratio Ra of the width W2 to the thickness (W2-W1)/2 is greater than 20.

12. Composition according to any one of Claims 2 to 11, **characterized in that** said spheres are capable of being obtained according to a process comprising:
(a) the introduction into an aqueous medium, in the presence of a hydrolysis catalyst and optionally a surfactant, of a compound (I) of formula SiX₄ and of a compound (II) of formula RSiY₃, where X and Y denote, independently of one another, a C₁-C₄ alkoxy group, an alkoxyethoxy group containing a C₁-C₄ alkoxy group, a C₂-C₄ acyloxy group, an N,N-dialkylamino group containing a C₁-C₄ alkyl group, a hydroxyl group, a halogen atom or a hydrogen atom, and R denotes an organic group having a carbon atom directly bonded to the silicon atom; and
(b) bringing the mixture resulting from step (a) into contact with an aqueous solution containing at least one polymerization catalyst and optionally at least one surfactant, at a temperature between 30 and 85°C, for at least two hours.

13. Composition according to Claim 12, **characterized in that** the compound of formula (I) is chosen from: tetramethoxysilane, tetraethoxysilane, tetrabutoxysilane, trimethoxyethoxysilane, tributoxyethoxysilane, tetraacetoxysilane, tetrapropioxysilane, tetra-(dimethylamino)silane, tetra(diethylamino)silane, silanetetraol, chlorosilanetriol, dichlorodisilanol, tetrachlorosilane and chlorotrihydrogenosilane.

14. Composition according to Claim 12 or 13, **characterized in that** the compound of formula (II) comprises a non-reactive organic group R and is chosen from: methyltrimethoxysilane, ethyltriethoxysilane, propyltributoxysilane, butyltributoxysilane, phenyltrimethoxyethoxysilane, methyltributoxyethoxysilane, methyltriacetoxysilane, methyltripropioxysilane, methyltri(dimethylamino)silane, methyltri(diethylamino)silane, methylsilanetriol, methylchlorodisilanol, methyltrichlorosilane and methyltrihydrogenosilane.

15. Composition according to Claim 12 or 13, **characterized in that** the compound of formula (II) comprises a reactive organic group R and is chosen from: silanes having an epoxy group; silanes having a (meth)acryloxy group; silanes having an alkenyl group; silanes having a mercapto group; silanes having an aminoalkyl group; silanes having a haloalkyl group; silanes having a glyceroxy group; silanes having a ureido group or silanes having a cyano group.

16. Composition according to any one of Claims 12 to 15, **characterized in that** X and/or Y denote a C₁-C₄ alkoxy group.

17. Composition according to Claim 12 or 16, **characterized in that** the compound (I) is tetraethoxysilane.

18. Composition according to Claim 12 or 16, **characterized in that** the compound (II) is methyltrimethoxysilane.

19. Composition according to any one of Claims 12 to 15, **characterized in that**, in step (a), the molar ratio of compound (I) to compound (II) ranges from. 30/70 to 50/50.

20. Composition according to Claim 19, **characterized in that** the molar ratio of compound (I) to compound (II) ranges from 35/65 to 45/55.

21. Composition according to Claim 19, **characterized in that** the molar ratio of compound (I) to compound (II) is 40/60.

22. Composition according to any one of Claims 12 to 21, **characterized in that** the weight ratio of water to the total of compounds (I) and (II) ranges from 10/90 to 70/30 in step (a).

23. Composition according to any one of Claims 12 to 22, **characterized in that** the hydrolysis and polymerization catalysts are independently chosen from sodium hydroxide, sodium carbonate, acetic acid, sodium dodecylbenzenesulphonate and hydrochloric acid.

24. Composition according to any one of Claims 12 to 23, **characterized in that** the surfactant is sodium dodecylbenzenesulphonate.

25. Composition according to any one of Claims 1 to 24, **characterized in that** the amount of sphere portions represents from 0.5 to 10% of the total weight of the composition.

26. Composition according to any one of Claims 1 to 25, **characterized in that** it is in the form of an O/W emulsion.

27. Composition according to any one of Claims 1 to 26, **characterized in that** it contains at least one volatile oil.

28. Composition according to Claim 27, **characterized in that** said volatile oil is chosen from: cyclic or linear silicones containing from 2 to 6 silicon atoms, branched hydrocarbons, volatile perfluoroalkanes and perfluoromorpholine derivatives.

29. Cosmetic method for fading out surface defects of the skin, comprising the topical application of the composition according to any one of Claims 1 to 28 to the skin.

30. Method according to Claim 29, **characterized in that** it is intended for reducing the shininess of the skin and/or for fading out pores.

31. Method according to Claim 29 or 30, **characterized in that** said skin is greasy or combination skin.

32. Method according to Claim 30 or 31, **characterized in that** the composition also contains at least one active agent chosen from: desquamating agents, anti-seborrhoeic agents, antimicrobial agents and calmatives.

33. Method according to Claim 29, **characterized in that** it is intended for fading out dark circles, marks, wrinkles and/or fine lines.

34. Method according to Claim 29 or 33, **characterized in that** said skin is aged skin.

35. Method according to Claim 33 or 34, **characterized in that** the composition also contains at least one active agent chosen from: desquamating agents or moisturizers; depigmenting or anti-pigmenting agents; anti-glycation agents; NO inhibitors; agents that stimulate the synthesis of dermal or epidermal macromolecules and/or that prevent their degradation; agents that stimulate the proliferation of fibroblasts or keratinocytes and/or the differentiation of keratinocytes; muscle relaxants or dermo-decontracting agents; free-radical scavengers or agents for combating pollution; tensioning agents; and agents that act on the microcirculation.

## Patentansprüche

1. Zusammensetzung in Form einer Emulsion, die in einem physiologisch akzeptablen Medium Teile von hohlen Kügelchen enthält, die aus einem Organosiliconmaterial bestehen, wobei die Kügelchen einen mittleren Durchmesser von 0,05 bis 10 µm aufweisen.

2. Zusammensetzung nach Anspruch 1, **dadurch gekennzeichnet, dass** das Siliconmaterial, aus dem die Teile von hohlen Kügelchen aufgebaut sind, ein vernetztes Polysiloxan mit dreidimensionaler Struktur ist, das aus Einheiten der Formel (I): SiO₂ und Einheiten der Formel (II): R¹SiO_{1,5} besteht, in der R¹ eine organische Gruppe bezeichnet, die ein direkt mit dem Siliciumatom verbundenes Kohlenstoffatom aufweist.

3. Zusammensetzung nach Anspruch 2, **dadurch gekennzeichnet, dass** R¹ eine nichtreaktive organische Gruppe darstellt, die unter den Gruppen Methyl, Ethyl, Propyl, Butyl oder Phenyl ausgewählt ist.

4. Zusammensetzung nach Anspruch 2, **dadurch gekennzeichnet, dass** R¹ eine reaktive organische Gruppe darstellt, die ausgewählt ist unter den Gruppen Epoxy, (Meth)acryloxy, Alkenyl, Mercaptoalkyl, Aminoalkyl, Halogenalkyl, Glyceroxy, Ureido, Cyano, die 2 bis 6 Kohlenstoffatome aufweisen.

5. Zusammensetzung nach Anspruch 3, **dadurch gekennzeichnet, dass** R¹ eine Methylgruppe darstellt.

6. Zusammensetzung nach einem der Ansprüche 1 bis 5, **dadurch gekennzeichnet, dass** die Teile von Kügelchen gebildet werden (im Längsschnitt) durch einen kleinen inneren Bogen (11), einen großen äußeren Bogen (21) und Segmente (31), welche die Enden der betreffenden Bögen miteinander verbinden, wobei die Weite (W1) zwischen den beiden Enden des kleinen inneren Bogens (11) im Mittel im Bereich von 0,01 bis 8 µm, die Weite (W2) zwischen den beiden Enden des großen äußeren Bogens (21) im Mittel im Bereich von 0,05 bis 10 µm und die Höhe (H) des großen äußeren Bogens (21) im Mittel im Bereich von 0,015 bis 8 µm liegen.

7. Zusammensetzung nach Anspruch 6, **dadurch gekennzeichnet, dass** die Segmente (31) eine Länge (W2-W1)/2 von 0,05 bis 1 µm aufweisen.

8. Zusammensetzung nach Anspruch 6 oder 7, **dadurch gekennzeichnet, dass** das Verhältnis Ra der Weite W2 zur Dicke (W2-W1)/2 mindestens gleich 3 ist.

9. Zusammensetzung nach Anspruch 8, **dadurch gekennzeichnet, dass** das Verhältnis Ra der Weite W2 zur Dicke (W2-W1)/2 größer als 5 ist.

10. Zusammensetzung nach Anspruch 9, **dadurch gekennzeichnet, dass** das Verhältnis Ra der Weite W2 zur Dicke (W2-W1)/2 größer als 10 ist.

11. Zusammensetzung nach Anspruch 10, **dadurch gekennzeichnet, dass** das Verhältnis Ra der Weite W2 zur Dicke (W2-W1)/2 größer als 20 ist.

12. Zusammensetzung nach einem der Ansprüche 2 bis 11, **dadurch gekennzeichnet, dass** die Kügelchen nach einem Verfahren erhältlich sind, das folgende Schritte aufweist:
(a) Einbringen einer Verbindung (I) der Formel SiX₄ und einer Verbindung (II) der Formel RSiY₃ in Gegenwart eines Hydrolysekatalysators und gegebenenfalls eines Tensids in ein wässeriges Medium, wobei bedeuten
X und Y unabhängig voneinander eine C₁-C₄-Alkoxygruppe, eine Alkoxyethoxygruppe mit einer C₁-C₄-Alkoxygruppe, eine C₂-C₄-Acyloxygruppe, eine N,N-Dialkylaminogruppe mit einer C₁-C₄-Alkylgruppe, eine Hydroxylgruppe, ein Halogenatom oder ein Wasserstoffatom
und R eine organische Gruppe, die ein direkt mit dem Siliciumatom verbundenes Kohlenstoffatom aufweist, sowie
(b) Inkontaktbringen des aus Schritt (a) resultierenden Gemisches mit einer wässerigen Lösung, die mindestens einen Polymerisationskatalysator und gegebenenfalls mindestens ein Tensid enthält, bei einer Temperatur im Bereich von 30 bis 85 °C während mindestens zwei Stunden.

13. Zusammensetzung nach Anspruch 12, **dadurch gekennzeichnet, dass** die Verbindung der Formel (I) ausgewählt ist unter Tetramethoxysilan, Tetraethoxysilan, Tetrabutoxysilan, Trimethoxyethoxysilan, Tributoxyethoxysilan, Tetraacetoxysilan, Tetrapropioxysilan, Tetra(dimethylamino)-silan, Tetra(diethylamino)-silan, Silantetraol, Chlorsilantriol, Dichlordisilanol, Tetrachlorsilan und Monochlorsilan.

14. Zusammensetzung nach Anspruch 12 oder 13, **dadurch gekennzeichnet, dass** die Verbindung der Formel (II) eine nichtreaktive organische Gruppe R aufweist und ausgewählt ist unter Methyltrimethoxysilan, Ethyltriethoxysilan, Propyltributoxysilan, Butyltributoxysilan, Phenyltrimethoxyethoxysilan, Methyltributoxyethoxysilan, Methyltriacetoxysilan, Methyltripropioxysilan, Methyltri(dimethylamino)-silan, Methyltri(diethylamino)-silan, Methylsilantriol, Methylchlordisilanol, Methyltrichlorsilan und Monomethylsilan.

15. Zusammensetzung nach Anspruch 12 oder 13, **dadurch gekennzeichnet, dass** die Verbindung der Formel (II) eine reaktive organische Gruppe R aufweist und ausgewählt ist unter Silanen mit einer Ethoxygruppe; Silanen mit einer (Meth)acryloxygruppe; Silanen mit einer Alkenylgruppe; Silanen mit einer Mercaptogruppe; Silanen mit einer Aminoalkylgruppe; Silanen mit einer Halogenalkylgruppe; Silanen mit einer Glyceroxygruppe; Silanen mit einer Ureidogruppe; Silanen mit einer Cyanogruppe.

16. Zusammensetzung nach einem der Ansprüche 12 bis 15, **dadurch gekennzeichnet, dass** X und/oder Y eine C₁-C₄-Alkoxygruppe bezeichnen.

17. Zusammensetzung nach Anspruch 12 oder 16, **dadurch gekennzeichnet, dass** die Verbindung (I) Tetraethoxysilan ist.

18. Zusammensetzung nach Anspruch 12 oder 16, **dadurch gekennzeichnet, dass** die Verbindung (II) Methyltrimethoxysilan ist.

19. Zusammensetzung nach einem der Ansprüche 12 bis 15, **dadurch gekennzeichnet, dass** in Schritt (a) das Molverhältnis von Verbindung (I) zu Verbindung (II) im Bereich von 30 : 70 bis 50 : 50 liegt.

20. Zusammensetzung nach Anspruch 19, **dadurch gekennzeichnet, dass** das Molverhältnis von Verbindung (I) zu Verbindung (II) im Bereich von 35 : 65 bis 45 : 55 liegt.

21. Zusammensetzung nach Anspruch 19, **dadurch gekennzeichnet, dass** das Molverhältnis von Verbindung (I) zu Verbindung (II) 40 : 60 beträgt.

22. Zusammensetzung nach einem der Ansprüche 12 bis 21, **dadurch gekennzeichnet, dass** das Gewichtsverhältnis des Wassers zur Gesamtmenge der Verbindungen (I) und (II) in Schritt (a) im Bereich von 10 : 90 bis 70 : 30 liegt.

23. Zusammensetzung nach einem der Ansprüche 12 bis 22, **dadurch gekennzeichnet, dass** der Hydrolysekatalysator und der Polymerisationskatalysator unabhängig ausgewählt sind unter Natriumhydroxid, Natriumcarbonat, Essigsäure, Natriumdodecylbenzolsulfonat und Salzsäure.

24. Zusammensetzung nach einem der Ansprüche 12 bis 23, **dadurch gekennzeichnet, dass** das Tensid Natriumdodecylbenzolsulfonat ist.

25. Zusammensetzung nach einem der Ansprüche 1 bis 24, **dadurch gekennzeichnet, dass** die Menge der Teile von Kügelchen 0,5 bis 10 % des Gesamtgewichts der Zusammensetzung beträgt.

26. Zusammensetzung nach einem der Ansprüche 1 bis 25, **dadurch gekennzeichnet, dass** sie in Form einer Öl-in-Wasser-Emulsion vorliegt.

27. Zusammensetzung nach einem der Ansprüche 1 bis 26, **dadurch gekennzeichnet, dass** sie mindestens ein flüchtiges Öl enthält.

28. Zusammensetzung nach Anspruch 27, **dadurch gekennzeichnet, dass** das flüchtige Öl ausgewählt ist unter cyclischen oder geradkettigen Siliconen mit 2 bis 6 Siliciumatomen, verzweigten Kohlenwasserstoffen, flüchtigen Perfluoralkanen und Perfluormorpholin-Derivaten.

29. Kosmetisches Verfahren zum Überdecken von Defekten der Hautoberfläche, das die topische Anwendung der Zusammensetzung nach einem der Ansprüche 1 bis 28 auf der Haut umfasst.

30. Verfahren nach Anspruch 29, **dadurch gekennzeichnet, dass** es dazu dient, Hautglanz zu verringern und/oder Poren abzudecken.

31. Verfahren nach Anspruch 29 oder 30, **dadurch gekennzeichnet, dass** es sich bei der Haut um eine Fetthaut oder eine Mischhaut handelt.

32. Verfahren nach Anspruch 30 oder 31, **dadurch gekennzeichnet, dass** die Zusammensetzung ferner mindestens einen Wirkstoff enthält, der ausgewählt ist unter Desquamationsmitteln, antiseborrhoischen Mitteln, antimikrobiellen Mitteln und reizlindernden Mitteln.

33. Verfahren nach Anspruch 29, **dadurch gekennzeichnet, dass** es dazu dient, Augenringe, Flecken, Falten und/oder Fältchen abzudecken.

34. Verfahren nach Anspruch 29 oder 33, **dadurch gekennzeichnet, dass** es sich bei der Haut um eine Altershaut handelt.

35. Verfahren nach Anspruch 33 oder 34, **dadurch gekennzeichnet, dass** die Zusammensetzung ferner mindestens einen Wirkstoff enthält, der ausgewählt ist unter Desquamationsmitteln oder Hydratationsmitteln; Depigmentierungsmitteln oder Antipigmentierungsmitteln; Antiglycationsmitteln; Anti-NO-Mitteln; Mitteln zur Stimulierung der Synthese von Makromolekülen der Dermis oder der Epidermis und/oder zur Verhinderung ihres Abbaus; Mitteln zur Stimulation der Proliferation von Fibroblasten oder Keratinocyten und/oder der Differenzierung von Keratinocyten; Myorelaxationsmitteln oder Mitteln zur Dermodekontraktion; Mitteln gegen freie Radikale oder umweltbedingte Verschmutzung; Mitteln zur Hautstraffung und Mitteln, die auf die Mikrozirkulation einwirken.
